# EUROPEAN PATENT SPECIFICATION

(11) **EP 2 288 344 B1**
(45) Date of publication and mention of the grant of the patent: **21.10.2015**
(21) Application number: 09757759.7
(22) Date of filing: 02.06.2009
(51) Int. Cl.: A61K 9/70, A61K 31/46, A61K 31/4745

(54) **TRANSDERMAL DRUG DELIVERY DEVICE**
TRANSDERMALE ARZNEIABGABEVORRICHTUNG
DISPOSITIF D'ADMINISTRATION DE MÉDICAMENT PAR VOIE TRANSDERMIQUE

(30) Priority: 02.06.2008 GB 0809905
(43) Date of publication of application: 02.03.2011
(73) Proprietor: The Secretary of State for Defence, Salisbury, Wiltshire SP4 0JQ (GB)
(72) Inventor: EASTERBROOK, Timothy, J., London SW1A 2HB (GB); GOSDEN, Edward, Surrey SM4 6QG (GB); MEYER, Elizabeth, 83714 Miesbach (DE)
(74) Representative: Farnsworth, Alastair Graham
(86) International application number: PCT/GB2009/001382
(87) International publication number: WO 2009/147387

(56) References cited:
- US-A- 5 089 267
- US-A- 5 939 095
- US-A1- 2004 010 054
- US-A1- 2006 183 796

## Description

The present invention is concerned with a transdermal drug delivery device for the pre-treatment and treatment of poisoning by highly toxic organophosphates as well as a method for its manufacture.

The threat posed by chemical warfare agents, and in particular, by nerve agents such as the highly toxic organophosphate compounds soman, sarin, tabun and agent VX has taken on new significance in recent times.

Present medical countermeasures are based in part on treatment with parasympathomimetic (cholinergic) compounds prior to exposure. For example, the present UK in-service nerve agent pre-treatment set (NAPS) relies on oral administration of pyridostigmine bromide prior to exposure and intramuscular injection of atropine, pralidoxime mesylate and avizafone following exposure.

The pre-treatment set is not particularly convenient to use and does not always prevent incapacitation on exposure to nerve agent so that the post-exposure treatment must often be undertaken by a second person. There has, therefore, been considerable interest in the development of new pre-treatments which are more fully protective against poisoning by highly toxic organophosphates.

The efficacy of physostigmine for the pre-treatment of nerve agent poisoning is well known but its use has been limited by its short half life and side effects such as nausea, vomiting and reduced cognitive function.

A number of animal studies have shown that sub-acute and acute injections of physostigmine and a parasympatholytic (anticholinergic) compound such as scopolamine or trihexyphenidyl can be more protective against nerve agent poisoning as compared to injection of physostigmine alone (Anderson, D.R. et al., in Drug Chem. Toxicol. 1991, 14(182), 1-19 and Lim, D.K. et al., in Pharmacol. Biochem. Behav., 1989, 31, 633-639).

Other animal studies have shown that sub-acute and acute injections of physostigmine and scopolamine or trihexyphenidyl are protective in guinea pigs for exposure to soman as compared to sub-acute injection of physostigmine with diminished side effect due to physostigmine (von Bredow, J. et al., in Fundam. Appl. Toxicol., 1991, 17, 782-789 and Lim, D.K. et al., ibid, 1991, 16, 482-489).

A long-acting transdermal system for the treatment of organophosphate poisoning (Levy, D. et al., in Proc. 3rd Int. Symp. Protection Against Chemical Warfare Agents, Umea, Sweden, 11-16 June 1989, Suppl.) consists of a physostigmine patch (or pad) and a scopolamine patch (Scopoderm®). The "two patch" system is shown to be protective in guinea pigs and pigs for exposure to 1.5 LD₅₀ soman.

The same system is reported to provide protection in pigs against 2.5 LD₅₀ soman at blood plasma concentration levels of physostigmine and scopolamine greater than 1.0 ng ml⁻¹ and 0.1 ng ml⁻¹ respectively (Levy, A et al., in Proc. 4th Int. Symp. Protection Against Chemical Warfare Agents, Stockholm, Sweden, 8-12 June 1992).

A human study in Alzheimer patients involving repeated application of physostigmine patches alone showed that a stable blood plasma concentration level which is protective in pigs can be achieved without adverse side effect other than skin irritation.

The transdermal therapeutic system is claimed to be protective in humans without side effect on the basis of "two patch" system studies in pigs showing protection at blood plasma concentration levels of physostigmine above 1 ng/ml and in humans showing the absence of nausea, vomiting and statistically significant changes in behaviour at blood plasma concentration levels of physostigmine of 0.3 ± 0.1 ng/ml.

EP 0 732 924 A1 is also concerned with a transdermal therapeutic system for the prophylaxis and/or treatment of poisoning by highly toxic organophophate compounds in humans. It discloses a "two patch" system and a "two-in-one" patch system which consists of a single patch containing a separate adhesive matrix for each of physostigmine and scopolamine.

These prior art transdermal therapeutic systems are, however, inconvenient to use and unsuitable for long lasting protection - especially of military and like personnel.

The scopolamine patch (Scopoderm®) of the "two patch" system must be worn behind the ear and other necessary, protective equipment can interfere with its adhesion.

Further, the skin contacting area of the "two-in-one patch" is so large that it cannot be located on a suitable part of human body for good adhesion and wearing properties without interference by other necessary, protective equipment.

A good adhesion is an acknowledged prerequisite for safe and effective operation of transdermal drug delivery systems (Wokovich, A.M. et al., Eur. J. Pharmaceut. Biopharmaceut., 2006, 64, 1-8).

The present invention generally aims to improve on this situation by providing a transdermal drug delivery device of smaller size and/or better adhesion.

The present inventors have surprisingly found that the improvement can be obtained by a transdermal drug delivery device in which both physostigmine and scopolamine are provided together in a single adhesive matrix comprising a neutral polyacrylate without functionality.

Accordingly, in one aspect, the present invention provides a transdermal drug delivery device for the pre-treatment and treatment of poisoning by highly toxic organophosphates, in the form of a matrix system comprising a backing layer, a drug containing adhesive layer and a release liner, in which the drug containing adhesive layer comprises a neutral polyacrylate without functionality having a uniform dispersion of a parasympathomimetic and a parasympatholytic therein, wherein the neutral polyacrylate without functionality is a polymer or a co-polymer of an ester of an acrylic acid which polymer or co-polymer is substantially free from any functional group capable of providing for cross-linking of the polymer or the co-polymer or for interaction with the parasympatho-mimetic or parasympatholytic.

Those skilled in the art will appreciate that the backing layer and the release liner will comprise a material which is impermeable to the components of the drug containing adhesive layer and that the material of the release liner will also permit its easy removal from the device.

As used herein, references to a drug containing adhesive layer having a uniform dispersion of a parasympathomimetic and a parasympatholytic therein are references to that layer having parasympathomimetic and parasympatholytic dissolved therein or to that layer including some or all of the parasympathomimetic and/or parasympatholytic in crystallize form.

As used herein, references to a neutral polyacrylate without functionality are references to a polymer or co-polymer of an ester of an acrylic acid which polymer or co-polymer is substantially free from any functional group capable of providing for cross-linking of the polymer or co-polymer or for interaction with the parasympathomimetic or parasympatholytic.

Such references will, in particular, be understood to be references to polyacrylates which are substantially free from carboxylic acid, amino and hydroxy functional groups.

In a preferred embodiment, the neutral polyacrylate without functionality comprises a co-polymer of 2-ethylhexyl acrylate and vinyl acetate, such as the commercially available Durotak® 4098.

Other suitable neutral polyacrylates without functionality include commercially available acrylate polymers Durotak® 9088 and Durotak® 9301 as well as the acrylate co-polymer Durotak® 901A.

The device may comprise a single matrix in which both actives are provided together but embodiments in which both actives are provided together in more than one matrix are also possible.

Suitable parasympathomimetics comprise indirect cholinesterase inhibitors, in particular, carbamates such as physostigmine, heptylphysostigmine, neostigmine, rivastigmine, pyridostigmine and demarcarium and physiologically acceptable salts thereof.

Other suitable indirect cholinesterase inhibitors include donepezil, edrophonium, galanthamine, tetrahydroacridin, velnacridine, taurine in optically pure or racemic form and physiologically acceptable salts thereof.

The parasympatholytic preferably comprises a tropane alkaloid in optically pure or racemic form or a physiologically acceptable salt thereof - but other parasympatholytics such as trihexyphenidyl, biperiden, profenamine, dexetimide, etanautine and orphenadrine may be used. Suitable tropane alkaloids include atropine, benzotropine, tropatepine and scopolamine.

The area weight of the drug containing adhesive layer and the % weight ratio of parasympathomimetic to parasympatholytic may be chosen in line with the therapeutic index of the actives to deliver long lasting (12 to 72 hours) blood plasma concentration levels of parasympathomimetic and parasympatholytic (ratio between 8:1 and 12:1 respectively) which are protective against poisoning by organophosphates and without side effects due to one or other the actives.

In one embodiment, the area weight of the drug containing adhesive layer is 100 g/m² or more. It may, for example, be between 100 to 500 g/m² and, in particular, between 100 and 350 g/m². In this embodiment, the % weight ratio of parasympatho-mimetic:parasympatholytic may be 30 to 50:1 or 35 to 45:1, for example, 40:1.

In a preferred embodiment, the drug containing adhesive layer includes a permeation enhancer.

Suitable permeation enhancers may be chosen from the group consisting of octylphenol, octylphenylether, propyleneglycol, polyethyleneglycol (PEG), in particular, PEG 400, fatty acids, in particular C₄ to C₂₂ straight chain fatty acids such as oleic acid, linoleic acid and adipic acid and fatty acid esters, in particular, isopropylmyristate, methyllaurate, glycerin monooleate and propylene glycol monooleate.

Advantageously, the permeation enhancer is also a tackifier. A suitable permeation enhancer which is also a tackifier is oleic acid.

The % weight of permeation enhancer in the drug containing adhesive layer may be generally chosen to help deliver protective blood plasma concentration levels of each drug and without side effects due to one or other of the drugs.

The % weight of permeation enhancer may, for example, be 2.5 to 15 %, in particular 5% to 10% of the drug containing adhesive layer.

In a preferred embodiment, in which the parasympathomimetic is physostigmine, the parasympatholytic is scopolamine and the permeation enhancer is oleic acid, the area weight of the drug containing adhesive layer is 300 g/m² and the % area weight ratio of physostigmine to scopolamine to oleic acid is respectively about 40:1:40.

In order to ensure good adhesion of the device to the skin, it is also preferred that the device includes an additional adhesive layer which circumferentially bounds the drug containing adhesive layer.

Advantageously, the additional adhesive layer has reduced flowability as compared to the drug containing adhesive layer, and is without cold flow.

The additional adhesive layer may be included as described in European patent 1 062 926 B1. It may, in particular, be provided by an overtape comprising a laminate of a high tack, acidic polyacrylate adhesive layer and a backing layer.

As used herein the expression "acidic polyacrylate" refers to a polymer or co-polymer of an ester of an acrylic acid which polymer or co-polymer comprises between 8 to 30 % weight of carboxylic acid functional group.

In one embodiment, the acidic polyacrylate adhesive is also a co-polymer of an ester of an acrylic acid and vinyl acetate, for example, Durotak® 2051.

It will be understood that in embodiments in which an overtape is provided, the backing layer of the overtape forms the backing layer for the device - and an intervening backing layer for the drug containing adhesive layer will be necessary.

Of course, the intervening backing layer will comprise a material which is impermeable to the components of the drug containing adhesive layer but will not circumferentially bound the drug containing adhesive layer.

In any case, the additional adhesive layer may have area weight below 50 g/m², in particular, between 20 g/m² to 40 g/m². In a preferred embodiment, in which the additional adhesive layer is provided by an overtape, the area weight of the adhesive layer is 25 g/m².

The area size of the overtape may be chosen to increase overall adhesion of the device to the skin - and may have a skin contacting width of between 1 mm and 10 mm.

In one embodiment, the skin contacting area of the drug containing adhesive layer is 21 cm² and the skin contacting width of the additional adhesive layer is 4 mm or 7 mm.

The release liner may be a conventional oversized type - and in embodiments including an overtape, it will be understood to contact both the drug containing and the additional adhesive layer. It may be a polyester or a polyethylene terephthalate liner, in particular, a transparent polyethylene terephthalate liner - such as Primeliner™ PET 1-s 50 µm.

In a preferred embodiment including overtape, the release liner is scored in a region thereon which overlays part of the additional adhesive layer whereby to provide a pull-off tag. The pull-off tag reduces the possibility of cold flow and contamination of persons or surfaces through or following contact with, the drug containing adhesive layer.

The backing layer and intervening backing layer may comprise a sheet or liner of a polyolefin, polyester, polyvinylidene chloride or a polyurethane material.

It has been found, however, that drug stability in the device is greatly improved by avoiding backing foils for the device which can admit light. It is preferred, therefore, that the backing foil is substantially impermeable to light.

In one embodiment, therefore, the backing layer comprises a laminate consisting of pigmented polyethylene and an aluminium vapour coated polyester such as that known by the name Scotchpak™ 1109.

The device may include a cover sheet for the backing foil and be provided with suitable protective packaging such as a sealed pouch (Climacraft). The cover sheet protects against the device sticking to its packaging as a result of sideward cold flow and preferably comprises a transparent polyethylene terephthalate sheet.

The transdermal drug delivery device of the present invention is much more convenient to use than the prior art transdermal systems - because the neutral poly-acrylate without functionality can support both actives in a single matrix and a higher mean flux of parasympathomimetic over a sustained period as compared to polyacrylates including carboxylic acid or alcohol functional groups.

The present invention provides a *single* patch - of much smaller size (39 cm² or less) than the "two-in-one" patch of the prior art - which delivers blood plasma concentration levels of parasympathomimetic and parasympatholytic which are protective against poisioning by highly toxic organophosphates and without side effect due to either drug (up to 72 hours).

A smaller patch size enables better adhesion - because the patch can be more easily located at sites on the human body providing for better adhesion and/or minimal interference by other protective equipment.

The neutral polyacrylate adhesive without functionality also provides for a device which is less likely to cold flow - as compared to the "two-in-one" patch of the prior art.

The device according to the present invention is suitable for prophylaxis against poisoning by chemical warfare agents such as sarin, sarum, tabun and agent VX as well as poisoning by organophosphate pesticides such as bromfenvinfos, chlorfenvinphos, crotoxyphos, dichlorvos, fospirate, naftalofos, phosphamidon and tetrachlorvinphos.

It may also be suitable for pre-treatment and treatment of poisoning by chemicals other than organophosphates which act on the central nervous system in a similar way i.e. by inhibition of AChE.

In a second aspect, the present invention provides a method for the manufacture of a transdermal drug delivery device for pre-treatment and treatment of poisoning by highly toxic organophosphates, comprising the steps of i) coating a first release liner and a second release liner with a solution comprising a neutral polyacrylate adhesive without functionality, a parasympathomimetic and a parasympatholytic ii) drying the coated release liners at a temperature between 50°C and 120°C whereby to obtain a first laminate and a second laminate each having a drug containing adhesive layer iii) applying a backing layer to the drug containing adhesive layer of the first laminate and removing the release liner therefrom whereby to obtain a third laminate and iv) fusing the the drug containing adhesive layer of the second laminate with the drug containing adhesive layer of the third laminate.

The drug containing adhesive solution may be obtained, for example, by the preliminary steps of adding an ethanolic solution of a parasympathomimetic and a parasympatholytic to a solution of a neutral polyacrylate without functionality and, optionally permeation enhancer, in ethyl acetate with mixing.

Of course, other solvents and temperatures may also be suitable and the amounts of each component of the solution will be chosen in the appropriate ratio having regard to the coating step i) and the desired area weight of the final drug containing adhesive layer.

In one embodiment, the coating step i) provides for coating the first and second release liners with the drug containing adhesive solution in two or more lanes, for example, in three or four lanes. Of course, the lanes on each liner should be appropriately positioned in relation to each other for the purposes of the fusing step iv).

In a preferred embodiment, the method provides that the first and second release liner are each coated with three lanes providing that the dried adhesive layers of the laminates obtained by step ii) each have an area weight of drug containing adhesive above 50 g/m² and contain parasympathomimetic and parasympatholytic in a % weight ratio of respectively 40:1.

The method may also comprise the further step of v) cutting the five layer laminate obtained from the fusing step so as to obtain one or more devices according to the present invention.

The cutting step v) may, in particular, provide that each device includes an oversize release liner or backing layer - but it is preferred that the oversize release liner is obtained by replacing the cut release liner.

As mentioned above, the device may also include an overtape. The method may, therefore, comprise the further steps of vi) providing a third release liner with a layer of an acidic polyacrylate adhesive whereby to obtain a fourth laminate, vii) applying a backing layer to the adhesive layer thereof viii) removing the release liner therefrom whereby to obtain an overtape and ix) adhering the overtape to the backing layer of a laminate cut from the laminate from step iv) so that it circumferentially bounds the drug containing adhesive layer.

In that case, the cutting step v) may provide for cutting of the excess backing layer from the five layer laminate so as to provide circumferential adhesion of the overtape around one or more laminates - and the method may include a further cutting step x) providing one or more finished devices.

Other embodiments of the method will be apparent from the foregoing description of the first aspect of the invention.

It will, in particular, be apparent that the method may use physostigmine as parasympathomimetic, scopolamine as parasympatholytic and oleic acid as permeation enhancer and provide that the first and second release liner are given one or more drug containing adhesive layers providing that the one or more dried layers on the laminates obtained by step ii) each have an area weight of drug containing adhesive above 150 g/m² and a % weight ratio of parasympathomimetic:parasympatholytic: permeation enhancer of respectively 40:1:40.

In a third aspect, the present invention provides for a kit of parts comprising at least one transdermal drug delivery device according to the first aspect of the invention in suitable protective packaging and a non-adhesive bandage, for example, a compression bandage, suitable for covering the whole of the device in use.

The present invention is now described with reference to the following drawings and Examples in which
Figure 1 is a cross-sectional view of a transdermal delivery device according to a preferred embodiment of the present invention;
Figure 2 shows a scheme illustrating a method for the manufacture of a transdermal delivery device according to a preferred embodiment of the present invention;
Figure 3 is a view of the skin contacting side of a transdermal device according to another preferred embodiment of the present invention; and
Figures 4 a), b) and c) are graphs showing respectively blood plasma concentration levles of physostigmine and of scopolamine as well as blood plasma AChE inhibition levels as a function of time for the device of Figure 3.

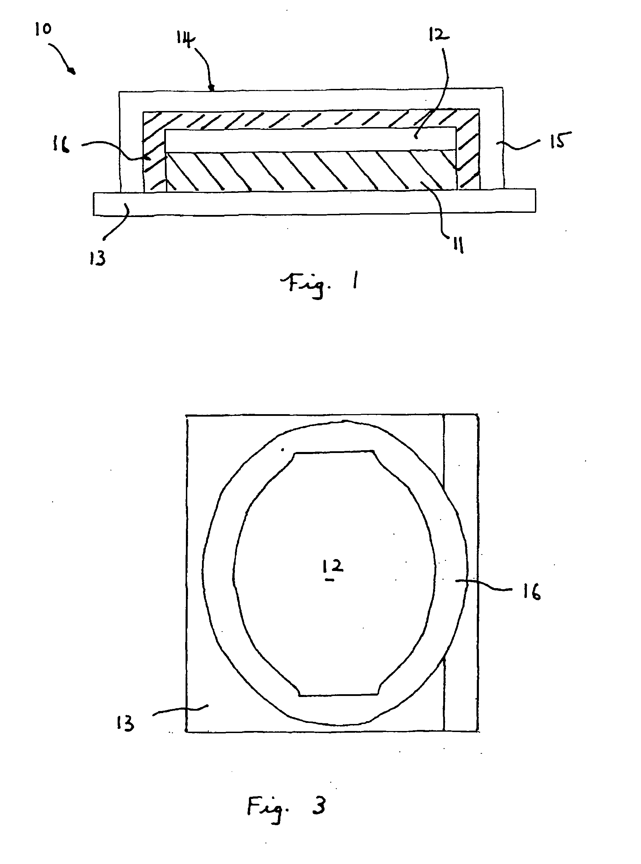

Referring now to Figure 1, a transdermal drug delivery device according to a preferred embodiment of the present invention comprises a rectangular patch, generally designated 10.

The patch comprises a single matrix 11 (hash line, downward left to right) of Durotak® 4098 adhesive containing dissolved physostigmine, scopolamine and oleic acid.

The skin contacting face of the adhesive matrix 11 contacts a removable release liner 13 and its opposite face contacts to an occlusive backing layer 12.

The device includes an overtape 14 in the form of a laminate comprising a backing foil 15 provided with a layer 16 of Durotak® 2051 adhesive (hash line, upward left to right) which overlays and circumferentially bounds the matrix 11 such that the adhesive layer 16 contacts the the backing layer 12 and the release liner 13 on faces which are opposite to the skin contacting face of the matrix.

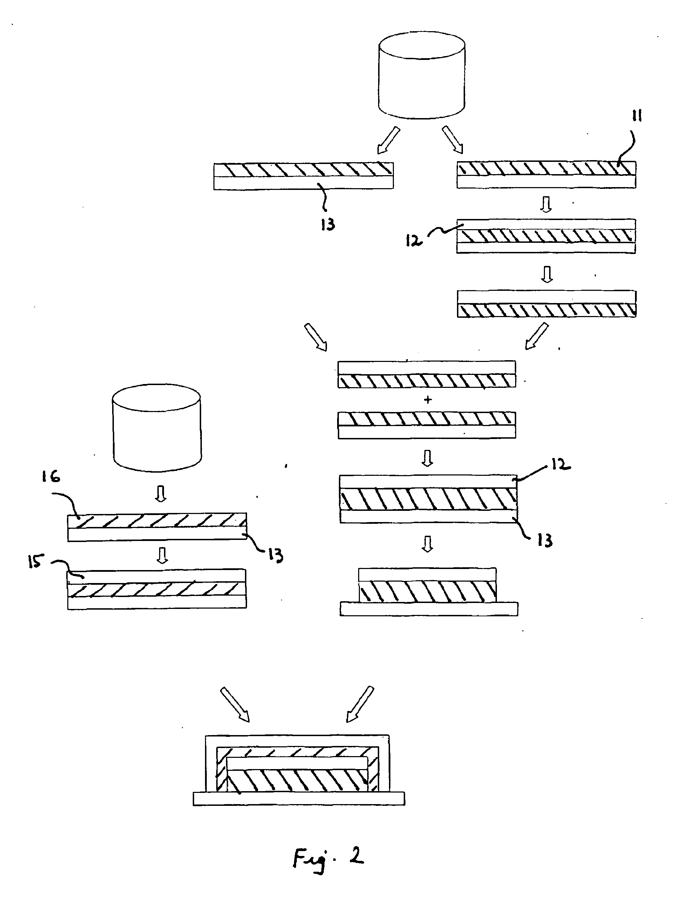

Referring now to Figure 2, the manufacture of the patch of Figure 1 involves the steps of adding an ethanolic solution of physostigmine and scopolamine to a solution of Durotak® 4098 and oleic acid with mixing.

The resultant solution is spread onto two separate continuous release sheets 13 in three corresponding lanes of equal amount. The release sheets are passed to an oven train for drying of the lanes at temperatures of 50°C, 75°C, 110°C and 120°C.

After cooling of the resultant laminates to room temperature, a backing liner 12 is adhered to the drug containing adhesive matrices of one laminate and its release sheet 13 is subsequently removed (right hand side of Figure 2).

The laminate without release sheet 13 is pressed into contact with the laminate without backing liner 12 to obtain a fused laminate in which a number of discrete matrices 11 are sandwiched between the backing liner 12 and release sheet 13.

The fused laminate is passed to a die cutter which cuts away excess backing liner from each matrix 11 so as to provide a number of discrete laminates including a continuous release liner 13.

Meanwhile, an overtape is prepared (left hand side of Figure 2) by spreading Durotak® 2051 onto a release liner 13 and passing the coated release liner to an oven train for drying at successive temperatures of 50°C, 75°C, 110°C and 120°C. After cooling to room temperature, a backing foil 15 is applied to the adhesive layer 16.

The overtape so obtained is pressed into contact with the laminates including the continuous release liner 13 so that it provides an additional adhesive layer 16 which circumferentially bounds each matrix 11 and contacts the release liner 13.

The laminate so obtained is passed to a die cutter which cuts out a number of devices with an oversize release liner 13.

Referring now to Figure 3, a transdermal drug delivery device according to another preferred embodiment of the invention comprises an oval patch generally designated 10 of substantially similar composition to the patch described in relation to Figure 1. However, the oval patch includes a transparent release liner 13 which is scored in a region overlaying part of the additional adhesive layer 16 so as to provide a pull-off tag 17 reducing the likelihood of cold flow and contamination from handling the device.

### Example 1

An oval patch of skin contacting area 34 cm² (or 39 cm²) contains a single matrix of area weight 300 g/m² and overtape of area weight 25 g/m² and has the following composition:
Matrix: skin contacting area 21 cm² (or 25 cm²):

| | | |
|---|---|---|
| Physostigmine free base | | 10 % by wt |
| Scopolamine free base | | 0.25 % by wt |
| Oleic acid | | 10 % by wt |
| Matrix adhesive: | Durotak® 4908 | 79.75 % by wt |
| Release liner: | Primeliner™ PET | 100 µm 1 -s |
| Backing layer: | Hostaphan SP MN15 | 15 µm |

Overtape: skin contacting width 7 mm

| | |
|---|---|
| Adhesive: | Durotak® 2051 |
| Backing foil: | Scotchpak™ 1109 |

A study of mean flux of physostigmine from patches containing a single matrix of similar area weight and drug composition (15% by weight physostigmine, 0.2% by weight scopolamine and 10% by weight oleic acid) was made in relation to a number of different polyacrylate adhesives in an *in vitro* (mouse) skin permeation model (Durrheim, H. et al. J. Pharm. Soc., 1980, 69(7), 781-786).

The model shows (Table 1) that a high flux is obtained for the neutral polyacrylates containing hydroxyl functional groups as compared to neutral polyacrylates without functional group (other than ester) - strongly suggesting the former as most suitable for delivering protective blood plasma concentration levels of physostigmine.

**Table 1:**

| Polyacrylate Adhesive | Mean flux rate |
|---|---|
| Durotak® 2510 | |
| - neutral, hydroxyl containing | 73 µg/cm²h |
| Durotak® 2287 | |
| - neutral, hydroxyl containing | 77 µg/cm²h |
| Durotak® 4098 | |
| - neutral, without functional group | 58 µg/cm²h |

However, a human study using the identical patches reveals the surprising result (Table 2) that blood plasma concentration levels of physostigmine are much higher in humans for neutral polyacrylates without functionality as compared to the neutral polyacrylates with hydroxyl functionality.

**Table 2:**

| Polyacrylate Adhesive | Concentration |
|---|---|
| Durotak® 2510 | |
| - neutral hydroxyl containing | 4.1 ng/ml |
| Durotak® 2287 | |
| - neutral hydroxyl containing | 2.1 ng/ml |
| Durotak® 4098 | |
| - without functionality | 8.9 ng/ml |

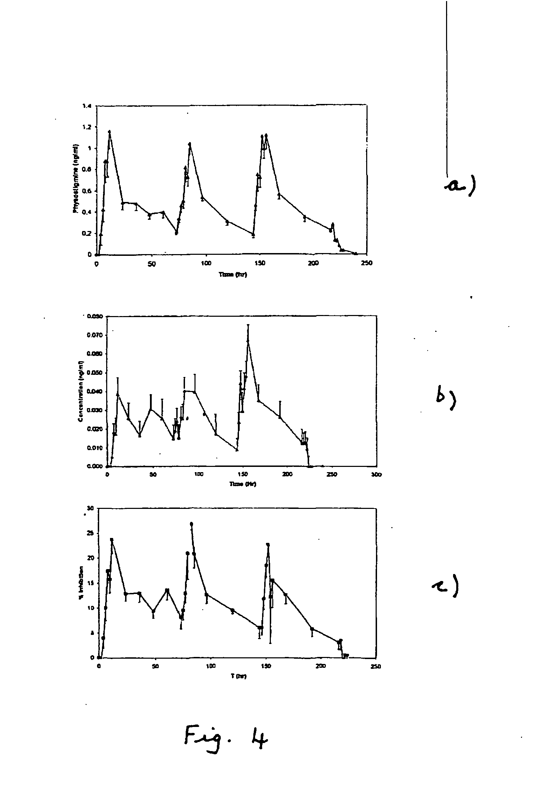

Figure 4 shows blood plasma concentration levels of physostigmine (a) and scopolamine (b) delived by the patch of Example 1 (repeated application every 72 hours). As may be seen high blood plasma concentration levels of physostigmine are rapidly obtained and maintained over about 24 h. Thereafter the concentration levels decrease to about half their highest value and gradually diminish over the remainder of the 72 hour period. The % blood plasma AChE inhibition (c) follows a similar course but remains at protective levels over the whole of the 72 hour period.

## Claims

1. A transdermal drug delivery device for the pre-treatment and treatment of poisoning by highly toxic organophosphates in humans, in the form of a matrix system comprising a backing layer, a drug containing adhesive layer and a release liner, in which the drug containing adhesive layer comprises a neutral polyacrylate without functionality having a uniform dispersion of a parasympathomimetic and a parasympatholytic therein, wherein the neutral polyacrylate without functionality is a polymer or a co-polymer of an ester of an acrylic acid which polymer or co-polymer is substantially free from any functional group capable of providing for cross-linking of the polymer or the co-polymer or for interaction with the parasympathomimetic or parasympatholytic.

2. A device according to Claim 1, in which the drug containing adhesive layer has an area weight above 100 g/m² and % weight ratio of parasympathomimetic to parasympatholytic about 40:1.

3. A device according to Claim 1 or Claim 2, in which the neutral polyacrylate without functionality is a co-polymer of 2-ethylhexyl acrylate and vinyl acetate.

4. A device according to any preceding Claim, in which the drug containing adhesive layer includes a permeation enhancer.

5. A device according to any preceding Claim, comprising an additional adhesive layer which circumferentially bounds the drug containing adhesive layer and has reduced flowability compared thereto, and is without cold flow.

6. A device according to Claim 5, in which the drug containing adhesive layer has area size about 21 cm² and the additional adhesive layer has area about 14 cm² and skin contacting width 7 mm.

7. A device according to Claim 6, in which the additional adhesive layer is provided by an overtape of area weight about 25 g/m².

8. A device according to any one of Claims 5 to 7, in which the additional adhesive layer comprises an acidic polyacrylate.

9. A device according to any one of Claims 5 to 8, in which the release liner is scored in a region of overlap with part of the additional adhesive layer whereby to provide a pull-off tag.

10. A device according to any preceding Claim, in which the backing layer is impermeable to light.

11. A device according to any preceding Claim, in which the parasympatho-mimetic is physostigmine or a physiologically acceptable salt thereof.

12. A device according to any preceding Claim, in which the parasympatholytic is scopolamine or a physiologically acceptable salt thereof.

13. A device according to any preceding Claim, in which the permeation enhancer is a fatty acid, for example, oleic acid.

14. A method for the manufacture of a transdermal drug delivery device according to any one of claims 1 to 13 for pre-treatment and treatment of poisoning by highly toxic organophosphates, comprising the steps of i) coating a first release liner and a second release liner with a solution comprising a neutral polyacrylate adhesive without functionality and a para-sympathomimetic and a parasympatholytic ii) drying the coated release liners at a temperature between 50°C and 120°C whereby to obtain a first laminate and a second laminate each having a drug containing adhesive layer iii) applying a backing layer to the drug containing adhesive layer of the first laminate and removing the release liner therefrom whereby to obtain a third laminate and iv) fusing the the drug containing adhesive layer of the second laminate with the drug containing adhesive layer of the third laminate.

15. A method according to Claim 14, comprising the further steps of vi) providing a third release liner with a layer of an acidic polyacrylate adhesive whereby to obtain a fourth laminate, vii) applying a backing layer to the adhesive layer viii) removing the release liner whereby to obtain an overtape and ix) adhering the overtape to a laminate obtained by cutting the laminate from step iv) so that it circumferentially bounds the drug containing adhesive layer.

16. A method according to any one of Claims 14 or Claim 15, in which the parasympathomimetic is physostigmine or a physiologically acceptable salt thereof.

17. A method according to any one of Claims 14 to 16, in which the parasympatholytic is scopolamine or a physiologically acceptable salt thereof.

18. A method according to any one of Claims 14 to 17, in which the permeation enhancer is oleic acid.

19. A kit of parts comprising at least one transdermal drug delivery device according to any one of Claims 1 to 13 in suitable protective packaging and a non-adhesive bandage, for example, a compression bandage, suitable for covering the whole of the device in use.

## Patentansprüche

1. Transdermales therapeutisches System für die Prophylaxe und für die Behandlung einer Vergiftung durch hochtoxische Organophosphate bei Menschen in der Form eines Matrixsystems, das eine Rückschicht, eine wirkstoffhaltige Haftschicht und eine Schutzfolie (Releaseliner) umfasst, wobei die wirkstoffhaltige Haftschicht ein neutrales Polyacrylat ohne funktionelle Gruppen aufweist, wobei darin ein Parasympathikomimetikum und ein Parasympathikolytikum gleichförmig dispergiert sind, wobei es sich bei dem neutralen Polyacrylat ohne funktionelle Gruppen um ein Polymer oder Copolymer eines Esters einer Acrylsäure handelt und wobei das Polymer oder Copolymer im Wesentlichen keine funktionellen Gruppen aufweist, die eine Vernetzung des Polymers oder Copolymers ermöglichen können oder die mit dem Parasympathikomimetikum oder Parasympathikolytikum wechselwirken können.

2. System nach Anspruch 1, wobei die wirkstoffhaltige Haftschicht ein Flächengewicht von 100 g/m² aufweist und das Gewichtsprozentverhältnis von Parasympathikomimetikum und Parasympathikolytikum etwa 40:1 beträgt.

3. System nach Anspruch 1 oder Anspruch 2, wobei es sich bei dem neutralen Polyacrylat ohne funktionelle Gruppen um ein 2-Ethylhexylacrylat/Vinylacetat-Copolymer handelt.

4. System nach einem der vorhergehenden Ansprüche, wobei die wirkstoffhaltige Haftschicht einen Permeationsverstärker aufweist.

5. System nach einem der vorhergehenden Ansprüche, das eine zusätzliche Haftschicht aufweist, die die wirkstoffhaltige Haftschicht umlaufend begrenzt und im Vergleich mit dieser eine geringere Fließfähigkeit aufweist und keinen kalten Fluss zeigt.

6. System nach Anspruch 5, wobei die wirkstoffhaltige Haftschicht eine Flächengröße von etwa 21 cm² besitzt und die zusätzliche Haftschicht eine Größe von etwa 14 cm² und eine in Hautkontakt befindliche Breite von 7 mm aufweist.

7. System nach Anspruch 6, wobei die zusätzliche Haftschicht in Form eines Deckpflasters (Overtape) mit einem Flächengewicht von etwa 25 g/m² vorliegt.

8. System nach einem der Ansprüche 5 bis 7, wobei die zusätzliche Haftschicht ein saures Polyacrylat umfasst.

9. System nach einem der Ansprüche 5 bis 8, wobei die Schutzfolie in einem Überlappungsbereich mit einem Teil der zusätzlichen Haftschicht eingekerbt ist, wodurch eine Abziehfahne geschaffen wird.

10. System nach einem der vorhergehenden Ansprüche, wobei die Rückschicht lichtundurchlässig ist.

11. System nach einem der vorhergehenden Ansprüche, wobei es sich bei dem Parasympathikomimetikum um Physostigmin oder ein physiologisch akzeptables Salz von Physostigmin handelt.

12. System nach einem der vorhergehenden Ansprüche, wobei es sich bei dem Parasympathikolytikum um Scopolamin oder ein physiologisch akzeptables Salz von Scopolamin handelt.

13. System nach einem der vorhergehenden Ansprüche, wobei es sich bei dem Penetrationsverstärker um eine Fettsäure, beispielsweise Ölsäure handelt.

14. Verfahren zur Herstellung eines transdermalen therapeutischen Systems nach einem der Ansprüche 1 bis 13 für die Prophylaxe und für die Behandlung einer Vergiftung durch hochtoxische Organophosphate, das die folgenden Schritte umfasst: i) Beschichten eines ersten Releaseliners und eines zweiten Releaseliners mit einer Lösung, die ein neutrales haftklebendes Polyacrylat ohne funktionelle Gruppen und ein Parasympathikomimetikum und ein Parasympathikolytikum enthält, ii) Trocknen der beschichteten Releaseliner bei einer Temperatur zwischen 50 und 120°C, um ein erstes Laminat und ein zweites Laminat zu bilden, die beide eine wirkstoffhaltige Haftschicht umfassen, iii) Aufbringen einer Rückschicht auf die wirkstoffhaltige Haftschicht des ersten Laminats und Entfernen des Releaseliners von diesem, um ein drittes Laminat zu bilden, und iv) Vereinigen der wirkstoffhaltigen Haftschicht des zweiten Laminats mit der wirkstoffhaltigen Haftschicht des dritten Laminats.

15. Verfahren nach Anspruch 14, das ferner die folgenden Schritte umfasst: vi) Bereitstellen eines dritten Releaseliners mit einer Schicht eines sauren haftklebenden Polyacrylats zur Bildung eines vierten Laminats, vii) Aufbringen einer Rückschicht auf die Haftschicht, viii) Entfernen des Releaseliners zur Bildung eines Overtapes und ix) haftendes Verbinden des Overtapes mit einem durch Schneiden des Laminats aus Schritt iv) erhaltenen Laminats, sodass es die wirkstoffhaltige Haftschicht umlaufend begrenzt.

16. Verfahren nach einem der Ansprüche 14 oder 15, worin es sich bei dem Parasympathikomimetikum um Physostigmin oder ein physiologisch akzeptables Salz von Physostigmin handelt.

17. Verfahren nach einem der Ansprüche 14 bis 16, worin es sich bei dem Parasympathikolytikum um Scopolamin oder ein physiologisch akzeptables Salz von Scopolamin handelt.

18. Verfahren nach einem der Ansprüche 14 bis 17, worin es sich bei dem Penetrationsverstärker um Ölsäure handelt.

19. Kit, das zumindest ein transdermales therapeutisches System nach einem der Ansprüche 1 bis 13 in einer geeigneten Schutzverpackung und eine nicht haftende Bandage, wie beispielsweise einen Kompressionsverband, umfasst, die im Gebrach das gesamte System abdecken kann.

## Revendications

1. Dispositif d'administration de médicament transdermique pour le pré-traitement et le traitement d'un empoisonnement par des organophosphorés hautement toxiques chez l'homme, sous la forme d'un système matriciel comprenant une couche de support, une couche adhésive contenant un médicament et une doublure détachable, où la couche adhésive contenant un médicament comprend un polyacrylate neutre sans fonctionnalité ayant une dispersion uniforme d'un parasympathomimétique et d'un parasympatholytique dedans, où le polyacrylate neutre sans fonctionnalité est un polymère ou un copolymère d'un ester d'un acide acrylique, lequel polymère ou copolymère est essentiellement exempt de n'importe quel groupe fonctionnel capable d'assurer la réticulation du polymère ou du copolymère ou l'interaction avec le parasympathomimétique ou le parasympatholytique.

2. Dispositif selon la revendication 1, dans lequel la couche adhésive contenant un médicament a un grammage supérieur à 100 g/m² et un rapport pondéral en pourcentage du parasympathomimétique sur le parasympatholytique d'environ 40:1.

3. Dispositif selon la revendication 1 ou 2, dans lequel le polyacrylate neutre sans fonctionnalité est un copolymère d'acrylate de 2-éthylhexyle et d'acétate de vinyle.

4. Dispositif selon l'une des revendications précédentes, dans lequel la couche adhésive contenant un médicament comporte un amplificateur de perméation.

5. Dispositif selon l'une des revendications précédentes, comprenant une couche adhésive supplémentaire qui délimite circonférentiellement la couche adhésive contenant un médicament et a une fluidité réduite par rapport à celle-ci, et ne présente pas de fluage à froid.

6. Dispositif selon la revendication 5, dans lequel la couche adhésive contenant un médicament a une superficie d'environ 21 cm² et la couche adhésive supplémentaire a une superficie d'environ 14 cm² et une largeur de contact avec la peau de 7 mm.

7. Dispositif selon la revendication 6, dans lequel la couche adhésive supplémentaire est fournie par une bande de recouvrement de grammage d'environ 25 g/m².

8. Dispositif selon l'une quelconque des revendications 5 à 7, dans lequel la couche adhésive supplémentaire comprend un polyacrylate acide.

9. Dispositif selon l'une quelconque des revendications 5 à 8, dans lequel la doublure détachable est marquée dans une région de chevauchement avec une partie de la couche adhésive supplémentaire de manière à fournir une étiquette d'enlèvement.

10. Dispositif selon l'une des revendications précédentes, dans lequel la couche de support est imperméable à la lumière

11. Dispositif selon l'une des revendications précédentes, dans lequel le parasympathomimétique est la physostigmine ou un sel physiologiquement acceptable de celle-ci.

12. Dispositif selon l'une des revendications précédentes, dans lequel le parasympatholytique est la scopolamine ou un sel physiologiquement acceptable de celle-ci.

13. Dispositif selon l'une des revendications précédentes, dans lequel l'amplificateur de perméation est un acide gras, par exemple l'acide oléique.

14. Procédé pour la fabrication d'un dispositif d'administration de médicament transdermique selon l'une quelconque des revendications 1 à 13 pour le pré-traitement et le traitement d'un empoisonnement par des organophosphorés hautement toxiques, comprenant les étapes qui consistent i) à revêtir une première doublure détachable et une deuxième doublure détachable avec une solution comprenant un adhésif polyacrylique neutre sans fonctionnalité et un parasympathomimétique et un parasympatholytique ii) à sécher les doublures détachables revêtues à une température comprise entre 50°C et 120°C de manière à obtenir un premier stratifié et un deuxième stratifié ayant chacun une couche adhésive contenant un médicament iii) à appliquer une couche de support à la couche adhésive contenant un médicament du premier stratifié et à retirer la doublure détachable de celui-ci de manière à obtenir un troisième stratifié et iv) à fusionner la couche adhésive contenant un médicament du deuxième stratifié avec la couche adhésive contenant un médicament du troisième stratifié.

15. Procédé selon la revendication 14, comprenant les étapes supplémentaires qui consistent vi) à fournir une troisième doublure détachable avec une couche d'un adhésif polyacrylique acide de manière à obtenir un quatrième stratifié, vii) à appliquer une couche de support à la couche adhésive viii) à retirer la doublure détachable de manière à obtenir une bande de recouvrement et ix) à faire adhérer la bande de recouvrement à un stratifié obtenu par découpage du stratifié de l'étape iv) de sorte qu'il délimite circonférentiellement la couche adhésive contenant un médicament.

16. Procédé selon l'une quelconque des revendications 14 ou 15, dans lequel le parasympathomimétique est la physostigmine ou un sel physiologiquement acceptable de celle-ci.

17. Procédé selon l'une quelconque des revendications 14 à 16, dans lequel le parasympatholytique est la scopolamine ou un sel physiologiquement acceptable de celle-ci.

18. Procédé selon l'une quelconque des revendications 14 à 17, dans lequel l'amplificateur de perméation est l'acide oléique.

19. Kit de parties comprenant au moins un dispositif d'administration de médicament transdermique selon l'une quelconque des revendications 1 à 13 dans un emballage de protection approprié et un bandage non adhésif, par exemple, un bandage de compression, apte à recouvrir l'ensemble du dispositif en cours d'utilisation.
